(19) Europäisches Patentamt
European Patent Office
Office européen des brevets

(11) EP 0 863 131 B1

(12) EUROPEAN PATENT SPECIFICATION

(45) Date of publication and mention of the grant of the patent:
**17.05.2000 Bulletin 2000/20**

(51) Int Cl.[7]: **C07C 233/49**, C08F 8/28, C08F 8/00

(21) Application number: **97103628.0**

(22) Date of filing: **05.03.1997**

# (54) Acrylic monomers, polymers thereof and crosslinking compositions

Acrylmonomeren, deren Polymere und Vernetzungszusammensetzungen

Monomères acryliques, polymères de ceux-ci, et composition réticulante

(84) Designated Contracting States:
**DE**

(43) Date of publication of application:
**09.09.1998 Bulletin 1998/37**

(73) Proprietor: **DAINIPPON INK AND CHEMICALS, INC.**
**Itabashi-ku Tokyo (JP)**

(72) Inventors:
- **Gaudl, Kai-Uwe, Dr.**
  **13467 Berlin (DE)**
- **Lachowicz, Artur, Dr.**
  **13467 Berlin (DE)**
- **Grahe, Gerwald, Dr.**
  **14195 Berlin (DE)**

(74) Representative: **Kraus, Walter, Dr. et al**
**Patentanwälte Kraus, Weisert & Partner**
**Thomas-Wimmer-Ring 15**
**80539 München (DE)**

(56) References cited:
**EP-A- 0 268 424**
**EP-A- 0 438 284**
**DE-A- 2 942 488**
**US-A- 4 360 425**

EP 0 863 131 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

## Description

The invention relates to new acrylic monomers and to polymers produced from these acrylic monomers. The invention also relates to crosslinking compositions containing the abovementioned polymers.

Synthetic resin bonding agents contain functional reactive groups which may be caused to react with other reactive groups in the bonding agent. In this way, the crosslinking composition is cured or crosslinked and obtains the desired properties, such as solvent resistance and hardness, for example. N-methylol(meth)acrylamides, which, when incorporated into polymers, are used as crosslinking agents, are used extensively as the reactive monomers in acrylic copolymers. A wealth of fields of application for acrylic resins containing N-methylol(meth)acrylamides is already known from the prior art, for example as general surface coatings [EP 0 438 284, JP 185 607, DE 29 43 030], antifouling coatings (B. Garg et al, Dep.Mater.Sci.Eng., CA88:7500p), aqueous adhesives [BE 854 393], protective coatings for concrete [FR 2 472 588], bonding agents for electrophoretic enamelling [DE 29 42 488] and aqueous coatings for automobiles [DE 2 709 308].

Furthermore, resins containing N-methylol(meth)acrylamides are used as coating or impregnating agents for textiles [US 4 075 387, CZ 171 319, JP 191 279 (1983), US 4 332 850]. In this way, the textiles are provided with better resistance to washing, dry-cleaning and mechanical wear.

N-methylol(meth)acrylamides are conventionally produced from (meth)acrylamide by the addition of formaldehyde.

Since this is an equilibrium reaction, formaldehyde may also be split off again. In the case of coating materials containing these monomers, it is known that they contain free formaldehyde [R.J. Skiscim, Celanese Polym.Sepc.Co. CA88:192608r] and that they additionally release formaldehyde when the coating materials are stored, applied or cured. This is particularly serious from the point of view of toxicology in the coating and impregnation of clothing textiles.

Various attempts have already been made to replace the formaldehyde with other less toxic aldehydes such as glyoxal [US 4 619 668] or dimethoxyacetaldehyde [Performance Chemicals, April/May 1994, pp. 41 to 46]. However, as a rule the ability of these systems to cure the coating is only slight, since the reactivity of these formaldehyde substitutes is less than that of formaldehyde, or undesired discolouration of the coating occurs, such as with N-(2,2-dimethoxy-1-hydroxy)ethylacrylamide [EP 0 612 718, page 3, line 23] for example. Other important properties of the coating, especially the solvent resistance with respect to organic solvents used in dry-cleaning are also negatively affected.

The object of the invention is to provide new acrylic monomers which may be converted into acrylic polymers by mono- or copolymerisation, from which acrylic polymers it is possible to produce curable formaldehyde-free bonding agents which may in particular be cured at low temperatures and which, in addition to exhibiting good adhesion to various substrates, are solvent resistant and do not yellow.

This object is achieved by the invention.

The present invention therefore provides compounds of the general formula I

$$CH_2{=}CR^1\text{-CO-NH-CH(O-CO-}R^3\text{)-CO-O-}R^2 \qquad \text{I}$$

wherein $R^1$ is a hydrogen atom or a methyl group and $R^2$ and $R^3$, independently of one another, are each a $C_1$-$C_4$ alkyl residue.

In the above general formula I, $R^1$ is a hydrogen atom or a methyl group. $R^2$ and $R^3$, independently of one another, are each a $C_1$-$C_4$ alkyl residue, for example a methyl, ethyl, propyl or butyl residue. The propyl and butyl residues may be straight-chain or branched-chain residues.

The invention also provides a method of producing the compounds of general formula I, which method is characterised in that a (meth)acrylamide of the general formula II

$$CH_2{=}CR^1\text{-CO-}NH_2 \qquad \text{II}$$

is reacted with a glyoxylic acid ester of the general formula III

$$OHC\text{-}COOR^2 \qquad \text{III}$$

and an anhydride of the general formula IV

$$(R^3\text{-CO})_2O \qquad IV$$

wherein $R^1$, $R^2$ and $R^3$ are as defined above.

The above reaction may be expressed by the following reaction equation 1:

$$CH_2{=}CR^1\text{-CO-NH}_2 + OHC\text{-COOR}^2 + (R^3\text{-CO})_2O \rightarrow$$

$$CH_2{=}CR^1\text{-CO-NH-CH(O-CO-R}^3\text{)-CO-O-R}^2 + R^3\text{-CO-OH} \qquad (1).$$

Glyoxylic acid methyl ester, glyoxylic acid ethyl ester and glyoxylic acid butyl ester are preferably used as the glyoxylic acid esters. Acetic anhydride and propionic acid anhydride are preferably used as the anhydrides.

The reaction temperatures are from 25 to 100°C, preferably from 40 to 70°C. During the reaction the starting material ratio acrylamide:glyoxylic acid ester:anhydride may range from 1:1:1 to 1:2:4 and preferably ranges from 1: 1.1:1.2 to 1:1.3:1.5. The reaction periods last from 1 to 4 hours, preferably 2 to 3 hours. All the reactants may be added together or in the sequence glyoxylic acid ester/anhydride: this depends on the reactivity of the individual starting materials in the individual case.

The reaction may be carried out in the absence of a solvent or in an inert solvent, such as, for example, toluene, xylene, ethyl acetate, butyl acetate or methyl ethyl ketone. To prevent a possible secondary reaction in the double bond, it is advantageous to add from 0.05 to 0.3 % of a polymerisation inhibitor, such as, for example, hydroquinone monomethylether or phenothiazine or a mixture thereof, and to conduct air into the reaction mixture during the reaction.

The reaction may be carried out without a catalyst. However, it is preferable to add a small amount of a protonic or Lewis acid, for example sulphuric acid, in the range of from 0.01 to 0.5 wt.% based on (meth)acrylamide.

When the reaction is complete the carboxylic acid formed as a by-product is removed by distillation under reduced pressure or washed out with a suitable solvent, for example water, or preferably with a saturated aqueous sodium hydrogen carbonate solution. The acrylic monomers according to the invention which are thus obtained are colourless, crystalline solids, which as a rule exhibit a degree of purity of over 90%. Further purification may be effected by recrystallisation from suitable solvents, such as butyl acetate or methyl ethyl ketone for example.

It may be considered surprising that the monomers according to the invention are formed simply from anhydride, glyoxylic acid ester and acrylamide, since it was in itself to be expected that anhydrides would also react directly with amides, causing acylation of the amide group [J. Chem. Soc. (C), 1968, 2779], which would inevitably lead to secondary reactions. This is not the case here.

Moreover, to produce the compounds of general formula I it is also possible to use derivatives of glyoxylic acid esters, such as glyoxylic acid alkyl ester-hemiacetals or -hydrates for example. The conditions of the reaction between (meth)acrylamides of general formula II and these derivatives are comparable with those of free aldehydes. Particularly suitable hemiacetals are glyoxylic acid methyl ester methyl-hemiacetal and glyoxylic acid butyl ester butyl-hemiacetal.

The invention therefore also provides a method of producing the compounds of general formula I, which is characterised in that a (meth)acrylamide of the general formula II

$$CH_2{=}CR^1\text{-CO-NH}_2 \qquad II$$

is reacted with a glyoxylic acid ester-hemiacetal of the general formula V

$$R^2\text{-O-CH(OH)-CO-OR}^2 \qquad V$$

and an anhydride of the general formula IV

$$(R^3\text{-CO})_2O \qquad IV$$

wherein $R^1$, $R^2$ and $R^3$ are as defined above.

This method may be expressed by the following reaction equation 2:

$$CH_2=CR^1\text{-CO-NH}_2 + R^2\text{-O-C(OH)-CO-OR}^2 + (R^3\text{-CO})_2O \rightarrow$$

$$CH_2=CR^1\text{-CO-NH-CH(O-CO-R}^3\text{)-CO-O-R}^2 + R^3\text{-CO-OH} + R^2\text{-OH} \qquad (2).$$

In this case it is advantageous to add twice the molar amount of anhydride, in order to recover as acetate the alcohol which arises, or to remove the alcohol which arises by distillation prior to the addition of anhydride or to carry out the reaction in two steps.

Hydrates of glyoxylic acid alkyl esters, such as glyoxylic acid methyl ester hydrate and glyoxylic acid butyl ester hydrate, may also be used as derivatives of glyoxylic acid esters, it being possible to produce such hydrates from methyl or butyl tartrate by oxidative C-C separation with periodic acid [L.D.M. Lolkema et al., Tetrahedron 50 (24), (1994) 7115-7128].

The invention therefore also provides a method of producing the compounds of general formula I, which is characterised in that a (meth)acrylamide of the general formula II

$$CH_2=CR^1\text{-CO-NH}_2 \qquad II$$

is reacted with a hydrate of a glyoxylic acid ester of the general formula IX

$$(HO)_2CH\text{-COOR}^2 \qquad IX$$

and an anhydride of the general formula IV

$$(R^3\text{-CO})_2O \qquad IV$$

wherein $R^1$, $R^2$ and $R^3$ are as defined above.

Moreover, it is also possible to carry out production of the compounds of general formula I in two stages, firstly producing a (meth)acrylamidoglycolic acid ester of general formula VI from a (meth)acrylamide and a glyoxylic acid ester (reaction equation 3).

$$CH_2=CR^1\text{-CO-NH}_2 + OHC\text{-CO-OR}^2 \rightarrow$$

$$CH_2=CR^{1-}\text{CO-NH-CH(OH)-CO-O-R}^2 \qquad VI \qquad (3).$$

The (meth)acrylamidoglycolic acid ester thus obtained is then further reacted with an anhydride of general formula IV (reaction equation 4):

$$CH_2=CR^1\text{-CO-NH-CH(OH)-CO-O-R}^2 + (R^3\text{-CO})_2O$$

$$\rightarrow CH_2=CR^1\text{-CO-NH-CH(O-CO-R}^3\text{)-CO-O-R}^2 + R^3\text{-CO-OH} \qquad IV \qquad (4).$$

The invention therefore also provides a method of producing compounds of the general formula I, which is characterised in that a (meth)acrylamide of the general formula II

$$CH_2=CR^1\text{-CO-NH}_2 \qquad II$$

is firstly reacted with a glyoxylic acid ester of the general formula III

$$OHC\text{-COOR}^2 \qquad III$$

to form a (meth)acrylamidoglycolic acid ester of the general formula VI

$$CH_2=CR^1\text{-CO-NH-CH(OH)-CO-O-}R^2 \qquad \text{VI}$$

and the (meth)acrylamidoglycolic acid ester obtained is reacted with an anhydride of the general formula IV

$$(R^3\text{-CO})_2O \qquad \text{IV,}$$

wherein $R^1$, $R^2$ and $R^3$ are as defined above, to form the desired compound.

Here too, surprisingly, no secondary reactions of the anhydride with the amide group were noted in the acrylamidoglycolates. Furthermore, it was also unforeseeable that the reaction between the acrylamidoglycolates of formula VI and the anhydrides would proceed under very mild reaction conditions within a few minutes, since the anhydrides as a rule only react with secondary alcohols at relatively high temperatures. The reaction may be carried out without a catalyst at from 40 to 50 °C or in the presence of small amounts of sulphuric acid (0.01 to 0.5 wt.%) at room temperature.

In the case of the abovedescribed additional variants of the method for producing the compounds of general formula I according to the invention, it is fundamentally possible to use those starting materials and reaction conditions which were described in conjunction with the production method described first.

The acrylic monomers of general formula I may be homopolymerised or they may be converted by copolymerisation with one or more comonomers into acrylic polymers of the general formula VII.

The invention therefore also provides a polymer of the general formula VII

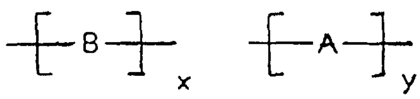

VII

wherein B signifies the repeating unit of the above-described acrylic monomer I and A signifies a different repeating unit from the group comprising acrylic and vinyl comonomers, wherein the proportion, indicated by x, of monomer B amounts to from 1 to 99 wt.% and the proportion, indicated by y, of monomer A amounts to from 99 to 1 wt.%, with the proviso that x + y = 100%.

Comonomers capable of polymerisation are, for example, methyl methacrylate, butyl acrylate, 2-hydroxyethyl acrylate, acrylamide, acrylonitrile, styrene or vinyl acetate, preferably methyl methacrylate, 2-hydroxyethyl acrylate, butyl acrylate and styrene.

The production of polymers of formula VII may be carried out in various ways. Methods involving bulk, mass, emulsion or solvent polymerisation or copolymerisation are particularly suitable. In the case of the production of polymers by means of solvent polymerisation, the known solvents are used. Particularly suitable solvents are hydrocarbons (heptane, toluene, xylene), esters (e.g. ethyl or butyl acetate, ethyl propionate), alcohols (e.g. propanol, butanol), ketones (e.g. 2-butanone, methyl isobutyl ketone), ethers (tetrahydrofuran, dioxan, ethylene glycol dialkylether, diethyleneglycol dialkylether), ethylene glycol alkyl ethers (e.g. ethylene glycol monomethylether, ethylene glycol monoethylether, ethylene glycol monobutylether, ethylene glycol dimethylether) or glycol ether esters (e.g. ethylene glycol monoethylether acetate, ethylene glycol dibutyl ether acetate, methoxypropyl acetate) as well as mixtures of the solvents listed.

Polymerisation may be initiated thermally or by radical formers. Particularly suitable polymerisation initiators are the known peroxide compounds, such as dialkyl peroxides, alkyl hydroperoxides and diacyl peroxides or esters of peroxycarboxylic acids, the known azoinitiators, such as, for example, azonitriles (e.g. azobisvaleronitrile, azobisdimethylvaleronitrile), azoamidines (e.g. azobismethylpropionamidine), azoamides (e.g. azobismethylpropionamide) or alkylazo compounds (e.g. azobistrimethylpentane) or the conventional redox initiators (e.g. persulphates, sodium hydrogen sulphite or iron (II) salts. The substances listed are known as polymerisation initiators (e.g. F. Runge and Taeger, "Einführung in die Chemie und Technologie der Kunststoffe" {"Introduction to Plastics Chemistry and Technology"}, Akademie Verlag Berlin, 1976).

Production of the polymers according to the invention may be effected within a broad temperature range of from 0 to 150°C, preferably from 40 to 90°C. The polymer thus obtained may be recovered by distilling off the solvent or by precipitation in homopolar solvents, such as petroleum benzine, hexane, cyclohexane, toluene and xylenes, and subsequent filtration or spray drying.

In the polymers according to the invention of the general formula VII the proportion, indicated by x, of acrylic monomer B amounts to from 1 to 99 wt.%, preferably from 3 to 30 wt.%, and the proportion, indicated by y, of monomer A amounts to from 99 to 1 wt.%, preferably from 97 to 70 wt.%, with the proviso that x + y = 100%. The molecular weight of the polymers according to the invention may be adjusted by known methods, especially by a regulator. Particularly suitable regulators are those on a mercaptan base, such as dodecyl mercaptan, tert.-butyl mercaptan, mercaptoalcohols and derivatives of mercaptoacetic and mercaptopropionic acid for example. The polymers obtained by solvent polymerisation have molecular weights of from 1,000 to 1,000,000, preferably from 10,000-100,000.

Production of the aqueous emulsions is effected by polymerisation of the respective monomers in an aqueous medium under the known conditions for emulsion copolymerisation. Particularly suitable polymerisation initiators are water-soluble peroxides, such as hydrogen peroxide, persulphates, such as sodium peroxide sulphate and potassium peroxodisulphate, or a mixture of these substances, especially in the presence of metal bisulphite. Moreover, accelerators may be added which contain soluble metal salts, the metallic component of which may occur in several valence states, such as iron (II) sulphate for example. Suitable emulsifiers are alkali metal salts of alkyl sulphonic acids, such as sodium dodecyl sulphonate, alkali metal salts of alkylaryl sulphonic acids, such as sodium dodecyl benzene sulphonate, or organic emulsifiers, such as ethoxylated alkyl phenols for example. The emulsion copolymerisation temperature ranges from 0 to 95°C, preferably from 40 to 90°C. The emulsion copolymers comprise molecular weights of from 10,000 to 1,000,000, preferably from 100,000 to 500,000. The solids content of the emulsions produced amounts to from 35 to 65%. Likewise, dispersions may also be produced by carrying out polymerisation in a low-boiling solvent miscible with water, water then being added and the solvent being distilled off.

The production of polymers of the general formula VII may also be effected by polymer-type reactions. To this end, first of all a polymer comprising amide side groups is produced using an amide monomer, such as acrylamide or methacrylamide for example, or a polymer comprising amidoglycolate side groups is produced using an acrylamidoglycolate, such as methyl acrylamidoglycolate.

The invention therefore also provides a method of producing polymers of the general formula VII which is characterised in that first of all a (meth)acrylamide of the general formula II

$$CH_2=CR^1\text{-CO-}NH_2 \qquad \text{II}$$

is polymerised with a further comonomer from the group comprising acrylic and vinyl monomers to form a polymer of the general formula VIII,

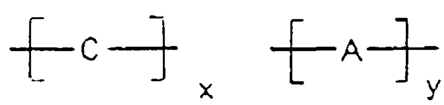

VIII

wherein C signifies the repeating unit of the (meth)acrylamide of the general formula II and A signifies a different repeating unit from the group comprising acrylic and vinyl monomers, wherein the proportion, indicated by x, of monomer C amounts to from 1 to 99 wt.% and the proportion, indicated by y, of monomer A amounts to from 99 to 1 wt.%, with the proviso that x + y = 100%, and the polymer thus obtained is then reacted with a glyoxylic acid ester of the general formula III

$$OHC\text{-}COOR^2 \qquad \text{III}$$

or a glyoxylic acid ester-hemiacetal of the general formula V

$$R^2\text{-O-CH(OH)-CO-OR}^2 \qquad V$$

or a glyoxylic acid ester-hydrate of the general formula IX

$$(HO)_2CH\text{-COOR}^2 \qquad IX$$

and an anhydride of the general formula IV

$$(R^3\text{-CO})_2O \qquad IV$$

wherein $R^1$, $R^2$ and $R^3$ are as defined above.

The invention also provides a method of producing polymers of the general formula VII which is characterised by the following stages:

1) reaction of a (meth)acrylamide of the general formula II

$$CH_2=CR^1\text{-CO-NH}_2 \qquad II$$

with a glyoxylic acid ester of the general formula III

$$OHC\text{-COOR}^2 \qquad III$$

to form a (meth)acrylamidoglycolic acid ester of the general formula VI

$$CH_2=CR^1\text{-CO-NH-CH(OH)-CO-O-R}^2 \qquad VI$$

2) copolymerisation of the (meth)acrylamidoglycolic acid ester obtained in stage 1) with a different comonomer from the group comprising acrylic and vinyl comonomers to form a poly(meth)acrylamidoglycolic acid ester of the general formula XI

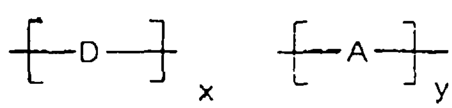

XI

wherein D signifies the repeating unit of the (meth)acrylamidoglycolic acid ester of the general formula VI and A signifies a different repeating unit from the group comprising acrylic and vinyl comonomers, wherein the proportion, indicated by x, of monomer D amounts to from 1 to 99 wt.% and the proportion, indicated by y, of monomer A amounts to from 99 to 1 wt.%, with the proviso that x + y = 100%, and

3) reaction of the poly(meth)acrylamidoglycolic acid ester thus obtained with an anhydride of the general formula IV

$$(R^3\text{-CO})_2O \qquad IV$$

wherein $R^1$, $R^2$ and $R^3$ are as defined above.

Production of these polymers is preferably effected by radical polymerisation as described above. In the case of poly(methacrylamides) the polymer solutions are reacted with a mixture of acetic anhydride/glyoxylic acid ester. In the case of acrylamidoglycolates, first of all a polymer is produced of the general formula XI

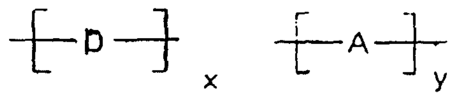

XI

wherein D represents the repeating units of the monomer of the general formula VI

$$CH_2=CR^1\text{-CO-NH-CH(OH)-CO-O-}R^2$$

and A represents the repeating units of one or more different acrylic or vinyl monomers,
and then reacted with acetic anhydride. The reaction conditions for these polymer-type reactions correspond substantially to those mentioned above for the corresponding monomers.

The invention also provides a cross-linking composition which comprises one or more polymers of the general formula VII

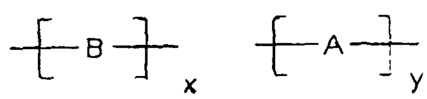

VII

wherein B signifies the repeating unit of the monomeric compound according to claim 1 and A signifies a different repeating unit from the group comprising acrylic and vinyl comonomers, wherein the proportion, indicated by x, of monomer B amounts to from 1 to 99 wt.% and the proportion, indicated by y, of monomer A amounts to from 99 to 1 wt.%, with the proviso that x + y = 100%, and optionally one or more conventional auxiliary agents and additives.

Thus, for the production of the crosslinking compositions it is possible to use copolymers of the general formula VII, which contain monomers of the general formula I as sole crosslinking component. The crosslinking composition is then self-crosslinking. However, the component A of the polymer of the general formula VII preferably contains a crosslinking partner comprising a functional group with active hydrogen. This may be an alcohol, urea, aminotriazine, carbamate, carboxylic acid or amide. Preferred crosslinking partners are alcohols which are incorporated as 2-hydroxyethyl-acrylate or -methacrylate into the polymers of the general formula VII as part of component A. However, it is also possible to use mixtures of copolymers of the general formula VII, which do not comprise any H-active crosslinking partners and additional monomeric, oligomeric or polymeric compounds, which comprise at least two H-active functional groups, as mentioned above.

The crosslinking compositions may be supplemented with regard to aspects of application technology by auxiliary agents and additives. Such agents may be solvents, fillers, colourants, pigments, plasticisers, flow-control agents, light-protecting agents, antioxidants, stabilisers, reactive thinners and neutralising substances. Suitable solvents are alcohols, ethylene glycol monoalkylethers, propylene glycol monoalkylethers, ethylene glycolether acetates, propylene glycol ether acetates, esters, ethers, aliphatic, cycloaliphatic, alkylaromatic and aromatic hydrocarbons, chlorinated hydrocarbons, ketones, alkylene carbonates, amides, sulphoxides and nitriles. Suitable fillers are, for example, kaolin, chalk, talc, quartz powder, slate flour and silicic acids. Titanium oxide, soot, lead chromate, iron oxide, phthalocyanine pigments and indigo are examples of suitable colourants or pigments. Suitable plasticisers are, for example, dioctyl phthalate, dibutyl sebacate, triphenyl phosphate etc.

Moreover, catalysts may also be used to accelerate curing of the crosslinking coating materials. As catalysts it is possible to use medium to strong organic and inorganic acids or Lewis acids, such as sulphuric acid, phosphoric acid, partially esterified phosphoric acids, hydrogen chloride, methane sulphonic acid, chlorosulphonic acid, benzene

sulphonic acid, p-toluene sulphonic acid, naphthol sulphonic acids, chloroacetic acids, oxalic acid and ethyl hexanoic acid, and as Lewis acid it is possible to use one or more substances from the group containing magnesium bromide, zinc chloride, zinc bromide, aluminium chloride, aluminium bromide, titanium tetrachloride, tin tetrachloride and boron trifluoride. The amount of catalyst added amounts to from 0.05 to 1.0 wt.% based on the solid copolymer. The aqueous emulsions produced do not as a rule require any catalyst. Curing of the resins is effected at from 25 to 160°C, preferably from 80 to 120°C within 30 minutes.

Curing, i.e. the crosslinking of the coating material, is tested by rubbing the cured surface with a cloth impregnated with methyl ethyl ketone as well as by determining the gel fraction, i.e. by extracting the soluble parts of the coating with acetone after curing. To this end, the cured coatings are placed in acetone for 24 hours, dried and the weight difference before and after extraction is determined. The gel fraction in % results from the original weight - the weight loss through extraction. The cured films as a rule exhibited a gel fraction of over 90% (see Tables 2 and 4, Examples 9 to 20, 24 to 35), i.e. only a slight weight loss and thus very good curing, which the person skilled in the art would not have foreseen.

Films which were cured in the range of from 80° to 120°C additionally exhibited good solvent resistance. This may be seen from Tables 2 and 4, Examples 9 to 20 and 24 to 35 through the abrasion tests carried out with MEK. The surfaces are not affected even after more than 70 to-and-fro strokes with MEK.

The invention will be described in more detail with the aid of the following exemplary embodiments.

**Examples:**

Examples 1-5: Production of monomers of general formula I

Example 1

Production of 2-acetoxy-2-acryloylamino-acetic acid methyl ester (MAAAG)

$$(CH_2{=}CH\text{-}CO\text{-}NH\text{-}CH(OCOCH_3)\text{-}COOCH_3)$$

7.10g (0.10 mol) of acrylamide, 11.0g (0.125 mol) of glyoxylic acid methyl ester (boiling point: 30-50°C, 15 mmHg), 12.0g (0.117 mol) of acetic anhydride and 50 mg of hydroquinone monomethylether are mixed and heated for 3 hours to T = 50°C. Thereafter, 50 mg of sulphuric acid is added and the mixture is allowed to cool over night, whereby the product crystallises. Small, tuft-like crystals form, which are filtered off and dried. Yield: 9.80 g, melting point: 79-81°C, 300-MHz-$^1$H-NMR (d$^6$-acetone): $\delta$ = 8.51 (1H, N-H), 6.61 (1H, d) 6.41 (m, 2H), 5.75 (1H, dd), 4.73 (3H, s), 2.06 (3H, s). 300-MHz-$^{13}$C-NMR (d$^6$-acetone): $\delta$ = 170.0 (C=O), 168.2 (C=O), 165.1 (C=O), 131.4 (C=C), 129.1 (C=C), 73.1 (C-H), 54.5 ($CH_3$), 20.1 ($CH_3$). FT-IR (KBr pellet): 3298 $\nu$(N-H), 3177, 3065 ($\nu$C-H), 1763, 1738, 1666 $\nu$(C=O), 1631 $\nu$(C=C), 1541 $\delta$(N-H).

Example 2

Production of 2-acetoxy-2-acryloylamino-acetic acid methyl ester (MAAAG)

$$(CH_2{=}CH\text{-}CO\text{-}NH\text{-}CH(OCOCH_3)\text{-}COOCH_3)$$

7.10g (0.10 mol) of acrylamide, 13.8g (0.115 mol) of glyoxylic acid methyl ester-hemiacetal, 25.0g (0.245 mol) of acetic anhydride and 50 mg of hydroquinone monomethylether are mixed and heated to T = 60°C for 3 hours, air being supplied thereto. Thereafter, 50 mg of sulphuric acid are added and the mixture is left to cool to room temperature. The volatile components are then distilled off under reduced pressure. The product remains as a colourless, solid residue. Yield: 9.02g. Melting point: 79°C.

Example 3

Production of 2-acetoxy-2-acryloylamino-acetic acid methyl ester (MAAAG)

$$(CH_2=CH-CO-NH-CH(OCOCH_3)-COOCH_3)$$

200.0g (1.25 mol) of acrylamidoglycolic acid methyl ester ($CH_2=CH-CO-NH-CH(OH)-COOCH_3$ are mixed with 150.0g (1.47 mol) of acetic anhydride and 0.25g of sulphuric acid and 0.50g of hydroquinone monomethylether. The mixture is heated to 40°C and after approx. 30 minutes a clear solution forms, which crystallises after cooling to room temperature. The raw product is washed with ethyl acetate and dried. Yield: 210.0g. Melting point: 80°C.

Example 4

Production of 2-acetoxy-2-acryloylamino-acetic acid butyl ester (BAAAG)

$$(CH_2=CH-CO-NH-CH(OCOCH_3)-COOC_4H_9)$$

7.10g (0.10 mol) of acrylamide, 14.6g (0.112 mol) of glyoxylic acid butyl ester, 25.0 g (0.245 mol) of acetic anhydride, 40 mg of sulphuric acid and 50 mg of hydroquinone monomethylether are mixed and heated to T = 55°C for 3 hours. Thereafter the mixture is allowed to cool over night and the volatile components are separated off at a reduced pressure. A yellowish oil remains, which slowly crystallises. Yield: 9.80g. 300-MHz-$^1$H-NMR ($d^6$-acetone): $\delta$ = 9.55 (1H, N-H), 6.41-6.2 (m, 2H), 5.75 (1H,dd), 4.08 (2H, t), 2.01 (3H, s), 1.63 (2H, m), 1.41 (2H, m), 0.93 (3H, t). 300-MHz-$^{13}$C-NMR ($d^6$-acetone): $\delta$ = 169.1 (C=O), 166.2 (C=O), 165.3 (C=O), 130.4 (C=C), 128.1 (C=C), 72.5 (C-H), 64.1 ($CH_2$), 50.3 ($CH_2$), 20.0 ($CH_3$), 19.3 ($CH_2$), 13.8 ($CH_3$).

Example 5

Production of 2-acetoxy-2-acryloylamino-acetic acid butyl ester (BAAAG)

$$(CH_2=CH-CO-NH-CH(OCOCH_3)-COOC_4H_9)$$

8.00g (0.04 mol) of acrylamidoglycolic acid butyl ester ($CH_2=CH-CO-NH-CH(OH)-COOC_4H_9$) are mixed with 5.10g (0.05 mol) acetic anhydride and 0.03g of sulphuric acid and 0.1g of hydroquinone monomethylether. The solid is dissolved and the mixture heated to 35°C. After cooling the product is crystallised out. The product is filtered off, washed with ligroin and air-dried. Yield: 9.2g, Melting point: 58-60°C.

Examples 6-7

Production of solvent-containing coating substances

An agitated vessel is filled with 100 parts of a 1:1 mixture of methyl ethyl ketone and butyl acetate and 0.5 parts of the initiator 2,2'azobis(2,4-dimethylvaleronitrile). Nitrogen is passed through the mixture, which is heated to T = 65°C. 100 parts of the respective monomeric compositions in Table 1 are then added dropwise over a period of 3 hours. Thereafter, another 0.1 parts of initiator are added and the mixture is stirred for a further 2 hours.

Table 1:

| Composition of the resins in wt.% | | | | | | | |
|---|---|---|---|---|---|---|---|
| Ex. | Resin No. | MAAAG | EHA | BMA | MMA | Solids [%] | Viscosity (Gardner) |
| 6 | 1 | 20.00 | 13.20 | 36.80 | 30.00 | 52.8 | U |
| 7 | 2 | 10.00 | 21.70 | 35.80 | 32.50 | 46.9 | S |

MAAAG = 2-acetoxy-2-acrylamino-acetic acid methyl ester,
EHA = ethylhexyl acrylate, BMA = butyl methacrylate, MMA = methyl methacrylate.

Example 8

Production of solvent-containing coating substances by polymer-type reaction

An agitated vessel is filled with 100 parts of a 50% solution of a methylacrylamidoglycolate resin in ethylene glycol monobutylether acetate with the composition (MAAG/MMA/BA/St = 10/35/35/20). Nitrogen is passed through the mixture, 15 parts of acetic anhydride are added and the mixture is heated to T = 75°C for 2 hours. Thereafter, the volatile components are distilled off and solvent optionally substituted. The resin obtained has a Gardener viscosity of T-U and a solids content of 50%.

Examples 9-20

Curing of the coatings

Resin solutions No. 1 and 2 of Examples 6 and 7 are mixed with the acrylic-resin (MMA/BA/HEMA = 45/35/25) in equimolar ratio of reactive groups and with different amounts of the catalyst p-toluene sulphonic acid, applied to test plates in layers 50µm thick and cured at room temperature or at a raised temperature, as shown in Table 2. Solvent resistance is tested by rubbing a cloth impregnated with methyl ethyl ketone (MEK) over the surface of the cured film until the film surface is destroyed and the degree of crosslinking is determined by the gel fraction.

Table 2:

| Example | Resin No. * | Crosslinking conditions | Catalyst** [wt. % based on solids] | Gel fraction [%] | MEK to-and-fro strokes | Yellowing |
|---|---|---|---|---|---|---|
| 9 | 1 | 25°C/7 days | 1.0 | 89 | <20 | no |
| 10 | 1 | 100°C/30 mins. | 0.25 | 94 | >75 | no |
| 11 | 1 | 100°C/30 mins. | 0.5 | 95 | >75 | no |
| 12 | 1 | 120°C/30 mins. | 0.1 | 94 | >75 | no |
| 13 | 1 | 140°C/30 mins. | - | 90 | >75 | no |
| 14 | 2 | 80°C/30 mins. | 0.5 | 88 | <25 | no |
| 15 | 2 | 100°C/30 mins. | 0.5 | 94 | >75 | no |
| 16 | 2 | 120°C/30 mins. | 0.25 | 95 | >75 | no |
| 17 | 2 | 25°C/7 days | 1.0 | 89 | <10 | no |
| 18 | 2 | 80°C/30 mins. | 0.25 | 81 | <25 | no |

* The curing mixtures consist of resin 1 or 2 from example 6 or 7 and the resin MMA/BA/HEMA = 45/35/25 as a 50 wt.% solution in MEK

** The catalyst is p-toluene sulphonic acid

Table 2: (continued)

| Example | Resin No. * | Crosslinking conditions | Catalyst** [wt. % based on solids] | Gel fraction [%] | MEK to-and-fro strokes | Yellowing |
|---|---|---|---|---|---|---|
| 19 | 2 | 140°C/30 mins. | 0.5 | 96 | >75 | no |
| 20 | 2 | 160°C/30 mins. | 0.1 | 95 | >75 | no |

* The curing mixtures consist of resin 1 or 2 from example 6 or 7 and the resin MMA/BA/HEMA = 45/35/25 as a 50 wt.% solution in MEK

** The catalyst is p-toluene sulphonic acid

Examples 21-23

Production of aqueous emulsions:

An agitated vessel is filled with 100 parts of demineralised water and 0.9 parts of sodium lauryl sulphate. Nitrogen is passed through the mixture, which is heated to T = 75°C. There follows metered addition of 10 parts of a monomeric composition such as in Examples 20-22 (Table 3) and 3 parts of a 2% sodium peroxodisulphate solution. After approximately 10-20 mins. the mixture takes on a bluish colour and 90 parts of the monomeric mixture and 27 parts of the 2% initiator solution are added dropwise at the same time over a period of 3 hours. The mixture is then cooled and filtered through a 100μm gauze. The copolymer lattices have Tg values in the range of 0-10°C and minimum film forming temperatures in the range of 0-20°C.

Table 3:

| Composition of the emulsions | | | | | | | | | |
|---|---|---|---|---|---|---|---|---|---|
| Ex. | Emulsion No. | MAAAG | MAA | BA | HEMA | MMA | EHA | Solids [%] | pH |
| 21 | 3 | 7.55 | 1.49 | 53.36 | 4.89 | 32.70 | | 35 | 2.4 |
| 22 | 4 | 6.64 | 1.40 | 53.41 | | 32.64 | 5.86 | 36 | 2.2 |
| 23 | 5 | 7.50 | | 37.50 | | 55.00 | | 40 | 2.5 |
| MAAAG = 2-acetoxy-2-acryloylamino-acetic acid methyl ester, EHA = ethylhexylacrylate, BA = butyl acrylate, HEMA = hydroxyethyl methacrylate, MMA = methylmethacrylate, MAA methacrylic acid. | | | | | | | | | |

Examples 24-35

Crosslinking of the aqueous emulsions

Emulsions Nos. 3-5 from Examples 21-23 are applied to glass plates, pre-dried for 1 hour at 25°C and cured at room temperature or a raised temperature. Solvent resistance is determined by rubbing with methyl ethyl ketone (MEK) until the film surface is destroyed and the degree of crosslinking is determined by the gel fraction.

Table 4:

| Example No. | Emulsion No. | Crosslinking conditions | Gel fraction [%] | MEK to-and-fro strokes | Yellowing |
|---|---|---|---|---|---|
| 24 | 3 | 25°C/7 days | 98 | <20 | no |
| 25 | 3 | 80°C/30 mins. | 94 | <25 | no |
| 26 | 3 | 100°C/30 mins. | 97 | >75 | no |
| 27 | 3 | 120°C/30 mins. | 99 | >75 | no |
| 28 | 4 | 25°C/7 days | 87 | <10 | no |
| 29 | 4 | 80°C/30 mins. | 86 | <15 | no |

Table 4: (continued)

| Example No. | Emulsion No. | Crosslinking conditions | Gel fraction [%] | MEK to-and-fro strokes | Yellowing |
|---|---|---|---|---|---|
| 30 | 4 | 100°C/30 mins | 94 | >55 | no |
| 31 | 4 | 120°C/30 mins. | 95 | >75 | no |
| 32 | 5 | 25°C/7 days | 81 | <10 | no |
| 33 | 5 | 80°C/30 mins. | 88 | <25 | no |
| 34 | 5 | 100°C/30 mins. | 91 | >65 | no |
| 35 | 5 | 120°C/30 mins. | 93 | >75 | no |

## Claims

**1.** Compounds of the general formula I

$$CH_2=CR^1\text{-CO-NH-CH(O-CO-}R^3\text{)-CO-O-}R^2 \qquad \text{I}$$

wherein $R^1$ is a hydrogen atom or a methyl group and $R^2$ and $R^3$, independently of one another, are each a $C_1$-$C_4$ alkyl residue.

**2.** A method of producing the compounds of general formula I according to claim 1, characterised in that a (meth) acrylamide of the general formula II

$$CH_2=CR^1\text{-CO-}NH_2 \qquad \text{II}$$

is reacted with a glyoxylic acid ester of the general formula III

$$OHC\text{-}COOR^2 \qquad \text{III}$$

and an anhydride of the general formula IV

$$(R^3\text{-CO})_2O \qquad \text{IV}$$

wherein $R^1$, $R^2$ and $R^3$ are as defined in claim 1.

**3.** A method of producing the compounds of general formula I according to claim 1, characterised in that a (meth) acrylamide of the general formula II

$$CH_2=CR^1\text{-CO-}NH_2 \qquad \text{II}$$

is reacted with a glyoxylic acid ester-hemiacetal of the general formula V

$$R^2\text{-O-CH(OH)-CO-O}R^2 \qquad \text{V}$$

and an anhydride of the general formula IV

$$(R^3\text{-CO})_2O \qquad \text{IV}$$

wherein $R^1$, $R^2$ and $R^3$ are as defined in claim 1.

**4.** A method of producing the compounds of general formula I according to claim 1, characterised in that a (meth) acrylamide of the general formula II

$$CH_2=CR^1\text{-CO-NH}_2 \qquad \text{II}$$

is reacted with a hydrate of a glyoxylic acid ester of the general formula IX

$$(HO)_2CH\text{-COOR}^2 \qquad \text{IX}$$

and an anhydride of the general formula IV

$$(R^3\text{-CO})_2O \qquad \text{IV}$$

wherein $R^1$, $R^2$ and $R^3$ are as defined in claim 1.

**5.** A method of producing compounds of the general formula I, characterised in that a (meth)acrylamide of the general formula II

$$CH_2=CR^1\text{-CO-NH}_2 \qquad \text{II}$$

is firstly reacted with a glyoxylic acid ester of the general formula III

$$OHC\text{-COOR}^2 \qquad \text{III}$$

to form a (meth)acrylamidoglycolic acid ester of the general formula VI

$$CH_2=CR^1\text{-CO-NH-CH(OH)-CO-O-R}^2 \qquad \text{VI}$$

and the (meth)acrylamidoglycolic acid ester obtained is reacted with an anhydride of the general formula IV

$$(R^3\text{-CO})_2O \qquad \text{IV,}$$

wherein $R^1$, $R^2$ and $R^3$ are as defined above, to form the desired compound.

**6.** A method according to any one of claims 2 to 5, characterised in that the reaction temperature is from 25 to 100°C.

**7.** A method according to any one of claims 2 to 6, characterised in that the reaction is carried out in the presence of an acidic catalyst.

**8.** A method according to claim 7, characterised in that the catalyst is selected from the group comprising protonic acids and Lewis acids.

**9.** A method according to claim 8, characterised in that the protonic acid is selected from the group comprising sulphuric acid, phosphoric acid, partially esterified phosphoric acids, hydrogen chloride, methane sulphonic acid, chlorosulphonic acid, benzene sulphonic acid, p-toluene sulphonic acid, naphthol sulphonic acids, chloroacetic acids, oxalic acid and ethyl hexanoic acid and as Lewis acid it is possible to use one or more substances from the group comprising magnesium bromide, zinc chloride, zinc bromide, aluminium chloride, aluminium bromide, titanium tetrachloride, tin tetrachloride and boron trifluoride.

**10.** A polymer of the general formula VII

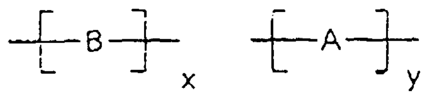

VII

wherein B signifies the repeating unit of the monomeric compound according to claim 1 and A signifies a different repeating unit from the group comprising acrylic and vinyl comonomers, wherein the proportion, indicated by x, of monomer B amounts to from 1 to 99 wt.% and the proportion, indicated by y, of monomer A amounts to from 99 to 1 wt.%, with the proviso that x + y = 100%.

**11.** A method of producing polymers according to claim 10, characterised in that a compound of the general formula I according to claim 1 is copolymerised with a different comonomer from the group comprising acrylic and vinyl comonomers.

**12.** A method of producing polymers according to claim 11, characterised in that the comonomer is selected from the group comprising methyl methacrylate, butyl acrylate, 2-hydroxyethyl acrylate, acrylamide, acrylonitrile, styrene or vinyl acetate.

**13.** A method of producing polymers according to claim 10, characterised in that first of all a (meth)acrylamide of the general formula II

$$CH_2=CR^1\text{-}CO\text{-}NH_2 \quad \text{II}$$

is polymerised with a further comonomer from the group comprising acrylic and vinyl monomers to form a polymer of the general formula VIII,

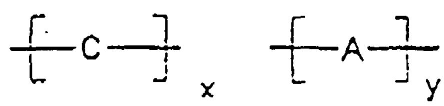

VIII

wherein C signifies the repeating unit of the (meth)acrylamide of the general formula II and A signifies a different repeating unit from the group comprising acrylic and vinyl monomers, wherein the proportion, indicated by x, of monomer C amounts to from 1 to 99 wt.% and the proportion, indicated by y, of monomer A amounts to from 99 to 1 wt.%, with the proviso that x + y = 100%,
and the polymer thus obtained is then reacted with a glyoxylic acid ester of the general formula III

$$OHC\text{-}COOR^2 \quad \text{III}$$

or a glyoxylic acid ester-hemiacetal of the general formula V

$$R^2\text{-}O\text{-}CH(OH)\text{-}CO\text{-}OR^2 \quad \text{V}$$

or a glyoxylic acid ester-hydrate of the general formula IX

$$(HO)_2CH\text{-}COOR^2 \quad \text{IX}$$

and an anhydride of the general formula IV

$$(R^3\text{-}CO)_2O \quad \text{IV}$$

wherein $R^1$, $R^2$ and $R^3$ are as defined in claim 1.

**14.** A method of producing polymers according to claim 10, characterised by the following stages:

1) reaction of a (meth)acrylamide of the general formula II

$$CH_2{=}CR^1\text{-}CO\text{-}NH_2 \quad \text{II}$$

with a glyoxylic acid ester of the general formula III

$$OHC\text{-}COOR^2 \quad \text{III}$$

to form a (meth)acrylamidoglycolic acid ester of the general formula VI

$$CH_2{=}CR^1\text{-}CO\text{-}NH\text{-}CH(OH)\text{-}CO\text{-}O\text{-}R^2 \quad \text{VI}$$

2) copolymerisation of the (meth)acrylamidoglycolic acid ester obtained in stage 1) with a different comonomer from the group comprising acrylic and vinyl comonomers to form a poly(meth)acrylamidoglycolic acid ester of the general formula XI

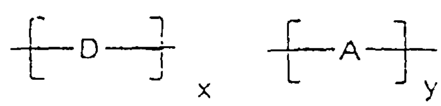

XI

wherein D signifies the repeating unit of the (meth)acrylamidoglycolic acid ester of the general formula VI and A signifies a different repeating unit from the group comprising acrylic and vinyl comonomers, wherein the proportion, indicated by x, of monomer D amounts to from 1 to 99 wt.% and the proportion, indicated by y, of monomer A amounts to from 99 to 1 wt.%, with the proviso that x + y = 100%, and

3) reaction of the poly(meth)acrylamidoglycolic acid ester thus obtained with an anhydride of the general formula IV

$$(R^3\text{-}CO)_2O \quad \text{IV}$$

wherein $R^1$, $R^2$ and $R^3$ are as defined in claim 1.

**15.** A method according to any one of claims 11 to 14, characterised in that the reaction temperature is from 50 to 100°C.

**16.** A method according to any one of claims 11 to 15, characterised in that the reaction is carried out in the presence of an acidic catalyst.

**17.** A method according to claim 16, characterised in that the catalyst is selected from the group comprising protonic acids and Lewis acids.

**18.** A method according to claim 17, characterised in that the protonic acid is selected from the group comprising sulphuric acid, phosphoric acid, partially esterified phosphoric acids, hydrogen chloride, methane sulphonic acid, chlorosulphonic acid, benzene sulphonic acid, p-toluene sulphonic acid, naphthol sulphonic acids, chloroacetic acids, oxalic acid and ethyl hexanoic acid, and as Lewis acid it is possible to use one or more substances from the group comprising magnesium bromide, zinc chloride, zinc bromide, aluminium chloride, aluminium bromide, titanium tetrachloride, tin tetrachloride and boron trifluoride.

**19.** A crosslinking composition, characterised in that it comprises one or more polymers according to claim 10 as well, optionally, as one or more conventional auxiliary agents and additives.

**20.** A crosslinking composition according to claim 19, characterised in that the polymer according to claim 10 contains H-active functional groups in the repeating units A of the general formula VII.

**21.** A crosslinking composition according to claim 19 or claim 20, characterised in that it additionally contains one or more monomeric, oligomeric or polymeric compounds containing at least two H-active functional groups.

**22.** A crosslinking composition according to claim 20 or claim 21, characterised in that the H-active functional group is derived from an alcohol, thioalcohol, amine, carboxylic acid amide, carboxylic acid, urea, triazine or urethane.

**23.** A crosslinking composition according to any one of claims 19 to 22, characterised in that it further comprises a solvent.

**24.** A crosslinking composition according to claim 23, characterised in that the solvent is selected from the group comprising water, alcohols, ethylene glycol monoalkylethers, propylene glycol monoalkylethers, ethylene glycol ether acetates, propylene glycol ether acetates, esters, ethers, aliphatic, cycloaliphatic, alkylaromatic and aromatic hydrocarbons, chlorinated hydrocarbons, ketones, alkylene carbonates, amides, sulphoxides and nitriles.

**25.** A crosslinking composition according to any one of claims 19 to 24, characterised in that it is in the form of an aqueous emulsion.

**26.** A crosslinking composition according to any one of claims 19 to 25, characterised in that it further comprises an acidic catalyst.

**27.** A crosslinking composition according to claim 26, characterised in that the catalyst is selected from the group comprising protonic acids and Lewis acids.

**28.** A crosslinking composition according to claim 27, characterised in that the protonic acid is selected from the group comprising sulphuric acid, phosphoric acid, partially esterified phosphoric acids, hydrogen chloride, methane sulphonic acid, chlorosulphonic acid, benzene sulphonic acid, p-toluene sulphonic acid, naphthol sulphonic acids, chloroacetic acids, oxalic acid and ethyl hexanoic acid, and as Lewis acid it is possible to use one or more substances from the group comprising magnesium bromide, zinc chloride, zinc bromide, aluminium chloride, aluminium bromide, titanium tetrachloride, tin tetrachloride and boron trifluoride.

**Patentansprüche**

**1.** Verbindungen der allgemeinen Formel I

$$CH_2=CR^1\text{-CO-NH-CH(O-CO-}R^3\text{)-CO-O-}R^2 \qquad \text{I}$$

worin $R^1$ für ein Wasserstoffatom oder eine Methylgruppe steht und $R^2$ und $R^3$ unabhängig voneinander jeweils für einen $C_1$-$C_4$ Alkylrest stehen.

**2.** Verfahren zur Herstellung der Verbindungen der allgemeinen Formel I nach Anspruch 1, dadurch **gekennzeichnet ,** daß ein (Meth)acrylamid der allgemeinen Formel II

$$CH_2=CR^1\text{-}CO\text{-}NH_2 \qquad II$$

mit einem Glyoxylsäurester der allgemeinen Formel III

$$OHC\text{-}COOR^2 \qquad III$$

und einem Anhydrid der allgemeinen Formel IV

$$(R^3\text{-}CO)_2O \qquad IV$$

worin $R^1$, $R^2$ und $R^3$ wie in Anspruch 1 definiert sind, umgesetzt wird.

**3.** Verfahren zur Herstellung der Verbindungen der allgemeinen Formel I nach Anspruch 1, dadurch **gekennzeichnet ,** daß ein (Meth)acrylamid der allgemeinen Formel II

$$CH_2=CR^1\text{-}CO\text{-}NH_2 \qquad II$$

mit einem Glyoxylsäureester-hemiacetal der allgemeinen Formel V

$$R^2\text{-}O\text{-}CH(OH)\text{-}CO\text{-}OR^2 \qquad V$$

und einem Anhydrid der allgemeinen Formel IV

$$(R^3\text{-}CO)_2O \qquad IV$$

worin $R^1$, $R^2$ und $R^3$ wie in Anspruch 1 definiert sind, umgesetzt wird.

**4.** Verfahren zur Herstellung der Verbindungen der allgemeinen Formel I nach Anspruch 1, dadurch **gekennzeichnet ,** daß ein (Meth)acrylamid der allgemeinen Formel II

$$CH_2=CR^1\text{-}CO\text{-}NH_2 \qquad II$$

mit einem Hydrat eines Glyoxylsäureesters der allgemeinen Formel IX

$$(HO)_2CH\text{-}COOR^2 \qquad IX$$

und einem Anhydrid der allgemeinen Formel IV

$$(R^3\text{-}CO)_2O \qquad IV$$

worin $R^1$, $R^2$ und $R^3$ wie in Anspruch 1 definiert sind, umgesetzt wird.

**5.** Verfahren zur Herstellung der Verbindungen der allgemeinen Formel I, dadurch **gekennzeichnet,** daß ein (Meth) acrylamid der allgemeinen Formel II

$$CH_2=CR^1\text{-}CO\text{-}NH_2 \qquad II$$

zuerst mit einem Glyoxylsäureester der allgemeinen Formel III

$$OHC\text{-}COOR^2 \qquad III$$

zur Bildung eines (Meth)acrylamidoglycolsäureesters der allgemeinen Formel VI

$$CH_2=CR^1\text{-}CO\text{-}NH\text{-}CH(OH)\text{-}CO\text{-}O\text{-}R^2 \qquad VI$$

umgesetzt wird und daß der erhaltene (Meth)acrylamidoglycolsäureester mit einem Anhydrid der allgemeinen Formel IV

$$(R^3\text{-}CO)_2O \qquad IV$$

worin $R^1$, $R^2$ und $R^3$ wie oben definiert sind, zur Bildung der gewünschten Verbindung umgesetzt wird.

**6.** Verfahren nach einem der Ansprüche 2 bis 5, dadurch **gekennzeichnet,** daß die Reaktionstemperatur 25 bis 100°C beträgt.

**7.** Verfahren nach einem der Ansprüche 2 bis 6, dadurch **gekennzeichnet,** daß die Reaktion in Gegenwart eines sauren Katalysators durchgeführt wird.

**8.** Verfahren nach Anspruch 7, dadurch **gekennzeichnet**, daß der Katalysator aus der Gruppe umfassend Protonensäuren und Lewis-Säuren ausgewählt wird.

**9.** Verfahren nach Anspruch 8, dadurch **gekennzeichnet,** daß die Protonensäure aus der Gruppe umfassend Schwefelsäure, Phosphorsäure, teilweise veresterte Phosphorsäuren, Chlorwasserstoff, Methansulfonsäure, Chlorsulfonsäure, Benzolsulfonsäure, p-Toluolsulfonsäure, Naphtholsulfonsäuren, Chloressigsäuren, Oxalsäure und Ethylhexansäure ausgewählt wird, und daß es möglich ist, als Lewis-Säure ein oder mehrere Substanzen aus der Gruppe umfassend Magnesiumbromid, Zinkchlorid, Zinkbromid, Aluminiumchlorid, Aluminiumbromid, Titantetrachlorid, Zinntetrachlorid und Bortrifluorid zu verwenden.

**10.** Polymeres der allgemeinen Formel VII

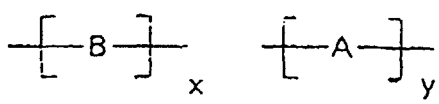

VII

worin B die Repetiereinheit der monomeren Verbindung nach Anspruch 1 angibt und A eine davon verschiedene Repetiereinheit aus der Gruppe umfassend Acryl- und Vinylcomonomere angibt, wobei die durch x angegebene Verhältnismenge des Monomeren B 1 bis 99 Gew.-% beträgt und die durch y angegebene Verhältnismenge des Monomeren A 99 bis 1 Gew.-% beträgt, mit der Maßgabe, daß x + y = 100%.

**11.** Verfahren zur Herstellung von Polymeren nach Anspruch 10, dadurch **gekennzeichnet,** daß eine Verbindung der allgemeinen Formel I nach Anspruch 1 mit einem davon unterschiedlichen Comonomeren aus der Gruppe umfassend Acryl- und Vinylcomonomeren copolymerisiert wird.

**12.** Verfahren zur Herstellung von Polymeren nach Anspruch 11, dadurch **gekennzeichnet,** daß das Comonomere

aus der Gruppe umfassend Methylmethacrylat, Butylacrylat, 2-Hydroxyethylacrylat, Acrylamid, Acrylnitril, Styrol oder Vinylacetat ausgewählt wird.

**13.** Verfahren zur Herstellung von Polymeren nach Anspruch 10, dadurch **gekennzeichnet,** daß zunächst ein (Meth) acrylamid der allgemeinen Formel II

$$CH_2=CR^1\text{-}CO\text{-}NH_2 \qquad II$$

mit einem weiteren Comonomeren aus der Gruppe umfassend Acryl- und Vinylmonomeren polymerisiert wird, um ein Polymeres der allgemeinen Formel VIII

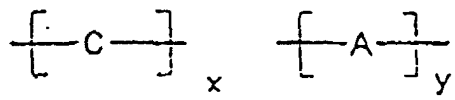

VIII

zu bilden, worin C die Repetiereinheit des (Meth)acrylamids der allgemeinen Formel II angibt und A eine davon verschiedene Repetiereinheit aus der Gruppe umfassend Acryl- und Vinylmonomere angibt, wobei die durch x angegebene Verhältnismenge des Monomeren C 1 bis 99 Gew.-% beträgt und die durch y angegebene Verhältnismenge des Monomeren A 99 bis 1 Gew.-% beträgt, mit der Maßgabe, daß x + y = 100%,
und daß das so erhaltene Polymere dann mit einem Glyoxylsäureester der allgemeinen Formel III

$$OHC\text{-}COOR^2 \qquad III$$

oder einem Glyoxylsäureester-hemiacetal der allgemeinen Formel V

$$R^2\text{-}O\text{-}CH(OH)\text{-}CO\text{-}OR^2 \qquad V$$

oder einem Glyoxylsäureester-hydrat der allgemeinen Formel IX

$$(HO)_2CH\text{-}COOR^2 \qquad IX$$

und einem Anhydrid der allgemeinen Formel IV

$$(R^3\text{-}CO)_2O \qquad IV$$

worin $R^1$, $R^2$ und $R^3$ wie in Anspruch 1 definiert sind, umgesetzt wird.

**14.** Verfahren zur Herstellung von Polymeren nach Anspruch 10, **gekennzeichnet** durch die folgenden Stufen:

1) Umsetzung eines (Meth)acrylamids der allgemeinen Formel II

$$CH_2=CR^1\text{-}CO\text{-}NH_2 \qquad II$$

mit einem Glyoxylsäureester der allgemeinen Formel III

$$OHC\text{-}COOR^2 \qquad III$$

zur Bildung eines (Meth)acrylamidoglycolsäureesters der allgemeinen Formel VI

$$CH_2{=}CR^1\text{-}CO\text{-}NH\text{-}CH(OH)\text{-}CO\text{-}O\text{-}R^2 \qquad VI$$

2) Copolymerisation des in Stufe 1) erhaltenen (Meth)acrylamidoglycolsäureesters mit einem davon verschiedenen Comonomeren aus der Gruppe umfassend Acryl- und Vinylcomonomere zur Bildung eines Poly(meth) acrylamidoglycolsäureesters der allgemeinen Formel XI

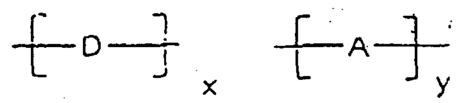

XI

worin D die Repetiereinheit des (Meth)acrylamidoglycolsäureesters der allgemeinen Formel VI angibt und A eine davon verschiedene Repetiereinheit aus der Gruppe umfassend Acryl- und Vinylcomonomere angibt, wobei die durch x angegebene Verhältnismenge des Monomeren D 1 bis 99 Gew.-% beträgt und die durch y angegebene Verhältnismenge des Monomeren A 99 bis 1 Gew.-% beträgt, mit der Maßgabe, daß x + y = 100%, und

3) Umsetzung des so erhaltenen Poly(meth)acrylamidoglycolsäureesters mit einem Anhydrid der allgemeinen Formel IV

$$(R^3\text{-}CO)_2O \qquad IV$$

worin $R^1$, $R^2$ und $R^3$ wie in Anspruch 1 definiert sind.

**15.** Verfahren nach einem der Ansprüche 11 bis 14, dadurch **gekennzeichnet,** daß die Reaktionstemperatur 50 bis 100°C beträgt.

**16.** Verfahren nach einem der Ansprüche 11 bis 15, dadurch **gekennzeichnet,** daß die Reaktion in Gegenwart eines sauren Katalysators durchgeführt wird.

**17.** Verfahren nach Anspruch 16, dadurch **gekennzeichnet,** daß der Katalysator aus der Gruppe umfassend Protonensäuren und Lewis-Säuren ausgewählt wird.

**18.** Verfahren nach Anspruch 17, dadurch **gekennzeichnet,** daß die Protonensäure aus der Gruppe umfassend Schwefelsäure, Phosphorsäure, teilweise veresterte Phosphorsäuren, Chlorwasserstoff, Methansulfonsäure, Chlorsulfonsäure, Benzolsulfonsäure, p-Toluolsulfonsäure, Naphtholsulfonsäuren, Chloressigsäuren, Oxalsäure und Ethylhexansäure ausgewählt wird, und daß es möglich ist, als Lewis-Säure ein oder mehrere Substanzen aus der Gruppe umfassend Magnesiumbromid, Zinkchlorid, Zinkbromid, Aluminiumchlorid, Aluminiumbromid, Titantetrachlorid, Zinntetrachlorid und Bortrifluorid zu verwenden.

**19.** Vernetzungsmasse, dadurch **gekennzeichnet,** daß sie ein oder mehrere Polymere nach Anspruch 10 sowie gegebenenfalls ein oder mehrere herkömmliche Hilfsmittel und Additive umfaßt.

**20.** Vernetzungsmasse nach Anspruch 19, dadurch **gekennzeichnet,** daß das Polymere nach Anspruch 10 H-aktive funktionelle Gruppen in den Repetiereinheiten A der allgemeinen Formel VII enthält.

**21.** Vernetzungsmasse nach Anspruch 19 oder 20, dadurch **gekennzeichnet,** daß sie zusätzlich ein oder mehrere

monomere, oligomere oder polymere Verbindungen, enthaltend mindestens zwei H-aktive funktionelle Gruppen, enthält.

**22.** Vernetzungsmasse nach Anspruch 20 oder 21, dadurch **gekennzeichnet**, daß sich die H-aktive funktionelle Gruppe von einem Alkohol, einem Thioalkohol, einem Amin, einem Carbonsäureamid, einer Carbonsäure, von Harnstoff, Triazin oder Urethan ableitet.

**23.** Vernetzungsmasse nach einem der Ansprüche 19 bis 22, dadurch **gekennzeichnet,** daß sie weiter ein Lösungsmittel umfaßt.

**24.** Vernetzungsmasse nach Anspruch 23, dadurch **gekennzeichnet,** daß das Lösungsmittel aus der Gruppe umfassend Wasser, Alkohole, Ethylenglycolmonoalkylether, Propylenglycolmonoalkylether, Ethylenglycoletheracetate, Propylenglycoletheracetate, Ester, Ether, aliphatische, cycloaliphatische, alkylaromatische und aromatische Kohlenwasserstoffe, chlorierte Kohlenwasserstoffe, Ketone, Alkylencarbonate, Amide, Sulfoxide und Nitrile ausgewählt ist.

**25.** Vernetzungsmasse nach einem der Ansprüche 19 bis 24, dadurch **gekennzeichnet,** daß sie in der Form einer wäßrigen Emulsion vorliegt.

**26.** Vernetzungsmasse nach einem der Ansprüche 19 bis 25, dadurch **gekennzeichnet,** daß sie weiterhin einen sauren Katalysator umfaßt.

**27.** Vernetzungsmasse nach Anspruch 26, dadurch **gekennzeichnet,** daß der Katalysator aus der Gruppe umfassend Protonensäuren und Lewis-Säuren ausgewählt ist.

**28.** Vernetzungsmasse nach Anspruch 27,dadurch **gekennzeichnet**, daß die Protonensäure aus der Gruppe umfassend Schwefelsäure, Phosphorsäure, teilweise veresterte Phosphorsäuren, Chlorwasserstoff, Methansulfonsäure, Chlorsulfonsäure, Benzolsulfonsäure, p-Toluolsulfonsäure, Naphtholsulfonsäuren, Chloressigsäuren, Oxalsäure und Ethylhexansäure ausgewählt wird, und daß es möglich ist, als Lewis-Säure ein oder mehrere Substanzen aus der Gruppe umfassend Magnesiumbromid, Zinkchlorid, Zinkbromid, Aluminiumchlorid, Aluminiumbromid, Titantetrachlorid, Zinntetrachlorid und Bortrifluorid zu verwenden.

**Revendications**

**1.** Composés de formule générale I :

$$CH_2=CR^1\text{-CO-NH-CH(O-CO-}R^3\text{)-CO-O-}R^2 \qquad \text{I}$$

où $R^1$ est un atome d'hydrogène ou un groupe méthyle et $R^2$ et $R^3$, indépendamment l'un de l'autre, sont chacun un résidu alkyle en $C_1$-$C_4$.

**2.** Procédé de production des composés de formule générale I selon la revendication 1, caractérisé en ce qu'un (méth)acrylamide de formule générale II

$$CH_2=CR^1\text{-CO-}NH_2 \qquad \text{II}$$

est mis à réagir avec un ester de l'acide glyoxylique de formule générale III

$$OHC\text{-}COOR^2 \qquad \text{III}$$

et avec un anhydride de formule générale IV

$$(R^3\text{-}CO)_2O \qquad IV$$

où $R^1$, $R^2$ et $R^3$ sont tels que définis dans la revendication 1.

**3.** Procédé de production des composés de formule générale I selon la revendication 1, caractérisé en ce qu'un (méth)acrylamide de formule générale II

$$CH_2{=}CR^1\text{-}CO\text{-}NH_2 \qquad II$$

est mis à réagir avec un hémiacétal d'ester d'acide glyoxylique de formule générale V

$$R^2\text{-}O\text{-}CH(OH)\text{-}CO\text{-}OR^2 \qquad V$$

et avec un anhydride de formule générale IV

$$(R^3\text{-}CO)_2O \qquad IV$$

où $R^1$, $R^2$ et $R^3$ sont tels que définis dans la revendication 1.

**4.** Procédé de production des composés de formule générale I selon la revendication 1, caractérisé en ce qu'un (méth)acrylamide de formule générale II

$$CH_2{=}CR^1\text{-}CO\text{-}NH_2 \qquad II$$

est mis à réagir avec un hydrate d'un ester d'acide glyoxylique de formule générale IX

$$(HO)_2CH\text{-}COOR^2 \qquad IX$$

et avec un anhydride de formule générale IV

$$(R^3\text{-}CO)_2O \qquad IV$$

où $R^1$, $R^2$ et $R^3$ sont tels que définis dans la revendication 1.

**5.** Procédé de production de composés de formule générale I, caractérisé en ce qu'un (méth)acrylamide de formule générale II

$$CH_2{=}CR^1\text{-}CO\text{-}NH_2 \qquad II$$

est tout d'abord mis à réagir avec un ester d'acide glyoxylique de formule générale III

$$OHC\text{-}COOR^2 \qquad III$$

pour former un ester d'acide (méth)acrylamidoglycolique de formule générale VI

$$CH_2{=}CR^1\text{-}CO\text{-}NH\text{-}CH(OH)\text{-}CO\text{-}O\text{-}R^2 \qquad VI$$

et l'ester d'acide (méth)acrylamidoglycolique obtenu est mis à réagir avec un anhydride de formule générale IV

$$(R^3\text{-CO})_2O \qquad IV$$

où $R^1$, $R^2$ et $R^3$ sont tels que définis ci-dessus, pour former le composé voulu.

**6.** Procédé selon l'une quelconque des revendications 2 à 5, caractérisé en ce que la température de réaction est de 25 à 100°C.

**7.** Procédé selon l'une quelconque des revendications 2 à 6, caractérisé en ce que la réaction est conduite en présence d'un catalyseur acide.

**8.** Procédé selon la revendication 7, caractérisé en ce que le catalyseur est choisi dans le groupe comprenant les acides protoniques et les acides de Lewis.

**9.** Procédé selon la revendication 8, caractérisé en ce que l'acide protonique est choisi dans le groupe comprenant l'acide sulfurique, l'acide phosphorique, les acides phosphoriques partiellement estérifiés, le chlorure d'hydrogène, l'acide méthanesulfonique, l'acide chlorosulfonique, l'acide benzènesulfonique, l'acide p-toluènesulfonique, les acides naphtolsulfoniques, les acides chloroacétiques, l'acide oxalique et l'acide éthylhexanoïque et comme acide de Lewis il est possible d'utiliser une ou plusieurs substances du groupe comprenant le bromure de magnésium, le chlorure de zinc, le bromure de zinc, le chlorure d'aluminium, le bromure d'aluminium, le tétrachlorure de titane, le tétrachlorure d'étain et le trifluorure de bore.

**10.** Polymère de formule générale VII

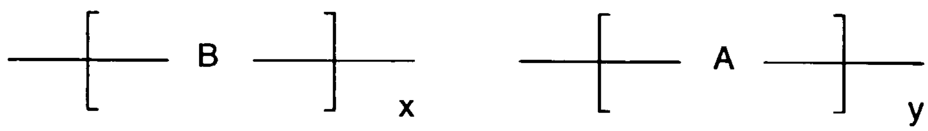

VII

où B désigne l'unité répétée du composé monomère selon la revendication 1 et A désigne une unité répétée différente du groupe comprenant les comonomères acryliques et vinyliques, où la proportion, indiquée par x, de monomère B est de 1 à 99 % en masse et la proportion, indiquée par y, de monomère A est de 99 à 1 % en masse, à condition que x + y = 100 %.

**11.** Procédé de production de polymères selon la revendication 10, caractérisé en ce qu'un composé de formule générale I selon la revendication 1 est copolymérisé avec un comonomère différent du groupe comprenant les comonomères acryliques et vinyliques.

**12.** Procédé de production de polymères selon la revendication 11, caractérisé en ce que le comonomère est choisi dans le groupe comprenant le méthacrylate de méthyle, l'acrylate de butyle, l'acrylate de 2-hydroxyéthyle, l'acrylamide, l'acrylonitrile, le styrène et l'acétate de vinyle.

**13.** Procédé de production de polymères selon la revendication 10, caractérisé en ce que, tout d'abord, un (méth) acrylamide de formule générale II

$$CH_2{=}CR^1\text{-CO-NH}_2 \qquad II$$

est polymérisé avec un autre comonomère du groupe comprenant les monomères acryliques et vinyliques pour former un polymère de formule générale VIII

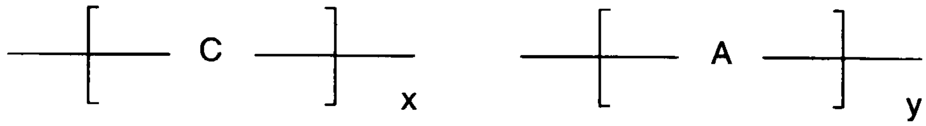

VIII

où C désigne l'unité répétée du (méth)acrylamide de formule générale II et A désigne une unité répétée différente du groupe comprenant les monomères acryliques et vinyliques, où la proportion, indiquée par x, de monomère C est de 1 à 99 % en masse et la proportion, indiquée par y, de monomère A est de 99 à 1 % en masse, à condition que x + y = 100 %,
et le polymère ainsi obtenu est ensuite mis à réagir avec un ester d'acide glyoxylique de formule générale III

$$OHC\text{-}COOR^2 \qquad III$$

ou avec un hémiacétal d'ester d'acide glyoxylique de formule générale V

$$R^2\text{-}O\text{-}CH(OH)\text{-}CO\text{-}OR^2 \qquad V$$

ou avec un hydrate d'ester d'acide glyoxylique de formule générale IX

$$(HO)_2CH\text{-}CCOR^2 \qquad IX$$

et avec un anhydride de formule générale IV

$$(R^3\text{-}CO)_2O \qquad IV$$

où $R^1$, $R^2$ et $R^3$ sont tels que définis dans la revendication 1.

**14.** Procédé de production de polymères selon la revendication 10, caractérisé par les étapes suivantes :

1) réaction d'un (méth)acrylamide de formule générale II

$$CH_2{=}CR^1\text{-}CO\text{-}NH_2 \qquad II$$

avec un ester d'acide glyoxylique de formule générale III

$$OHC\text{-}COOR^2 \qquad III$$

pour former un ester d'acide (méth)acrylamidoglycolique de formule générale VI

$$CH_2{=}CR^1\text{-}CO\text{-}NH\text{-}CH(OH)\text{-}CO\text{-}O\text{-}R^2 \qquad VI$$

2) copolymérisation de l'ester d'acide (méth)acrylamidoglycolique obtenu dans l'étape 1) avec un comonomère différent du groupe comprenant les comonomères acryliques et vinyliques pour former un poly(ester d'acide (méth)acrylamidoglycolique) de formule générale XI

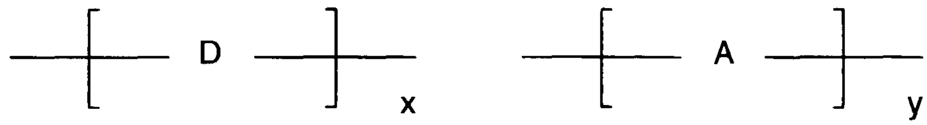

XI

où D désigne l'unité répétée de l'ester d'acide (méth)acrylamidoglycolique de formule générale VI et A désigne une unité répétée différente du groupe comprenant les comonomères acryliques et vinyliques, où la proportion, indiquée par x, de monomère D est de 1 à 99 % en masse et la proportion, indiquée par y, de monomère A est de 99 à 1 % en masse, à condition que x + y = 100 %, et

3) réaction du poly(ester d'acide (méth)acrylamidoglycolique) ainsi obtenu avec un anhydride de formule générale IV

$$(R^3\text{-CO})_2O \qquad \text{IV}$$

où $R^1$, $R^2$ et $R^3$ sont tels que définis dans la revendication 1.

**15.** Procédé selon l'une quelconque des revendications 11 à 14, caractérisé en ce que la température de réaction est de 50 à 100°C.

**16.** Procédé selon l'une quelconque des revendications 11 à 15, caractérisé en ce que la réaction est conduite en présence d'un catalyseur acide.

**17.** Procédé selon la revendication 16, caractérisé en ce que le catalyseur est choisi dans le groupe comprenant les acides protoniques et les acides de Lewis.

**18.** Procédé selon la revendication 17, caractérisé en ce que l'acide protonique est choisi dans le groupe comprenant l'acide sulfurique, l'acide phosphorique, les acides phosphoriques partiellement estérifiés, le chlorure d'hydrogène, l'acide méthanesulfonique, l'acide chlorosulfonique, l'acide benzènesulfonique, l'acide p-toluènesulfonique, les acides naphtolsulfoniques, les acides chloroacétiques, l'acide oxalique et l'acide éthylhexanoïque, et comme acide de Lewis il est possible d'utiliser une ou plusieurs substrances du groupe comprenant le bromure de magnésium, le chlorure de zinc, le bromure de zinc, le chlorure d'aluminium, le bromure d'aluminium, le tétrachlorure de titane, le tétrachlorure d'étain et le trifluorure de bore.

**19.** Composition réticulante, caractérisée en ce qu'elle comprend un ou plusieurs polymères selon la revendication 10 et, éventuellement, un ou plusieurs agents auxiliaires et additifs conventionnels.

**20.** Composition réticulante selon la revendication 19, caractérisée en ce que le polymère selon la revendication 10 contient des groupes fonctionnels H-actifs dans les unités répétées A de la formule générale VII.

**21.** Composition réticulante selon la revendication 19 ou la revendication 20, caractérisée en ce qu'elle contient en outre un ou plusieurs composés monomères, oligomères ou polymères contenant au moins deux groupes fonctionnels H-actifs.

**22.** Composition réticulante selon la revendication 20 ou la revendication 21, caractérisée en ce que le groupe fonctionnel H-actif est dérivé d'un alcool, d'un thioalcool, d'une amine, d'un amide d'acide carboxylique, d'un acide carboxylique, d'une urée, d'une triazine ou d'un uréthane.

**23.** Composition réticulante selon l'une quelconque des revendications 19 à 22, caractérisée en ce qu'elle comprend en outre un solvant.

**24.** Composition réticulante selon la revendication 23, caractérisée en ce que le solvant est choisi dans le groupe comprenant l'eau, les alcools, les monoalkyléthers d'éthylèneglycol, les monoalkyléthers de propylèneglycol, les acétates d'éthers d'éthylèneglycol, les acétates d'éthers de propylèneglycol, les esters, les éthers, les hydrocarbures aliphatiques, cycloaliphatiques, alkylaromatiques et aromatiques, les hydrocarbures chlorés, les cétones, les carbonates d'alkylène, les amides, les sulfoxydes et les nitriles.

**25.** Composition réticulante selon l'une quelconque des revendications 19 à 24, caractérisée en ce qu'elle est sous forme d'une émulsion aqueuse.

**26.** Composition réticulante selon l'une quelconque des revendications 19 à 25, caractérisée en ce qu'elle comprend en outre un catalyseur acide.

**27.** Composition réticulante selon la revendication 26, caractérisée en ce que le catalyseur est choisi dans le groupe comprenant les acides protoniques et les acides de Lewis.

**28.** Composition réticulante selon la revendication 27, caractérisée en ce que l'acide protonique est choisi dans le groupe comprenant l'acide sulfurique, l'acide phosphorique, les acides phosphoriques partiellement estérifiés, le chlorure d'hydrogène, l'acide méthanesulfonique, l'acide chlorosulfonique, l'acide benzènesulfonique, l'acide p-toluènesulfonique, les acides naphtolsulfoniques, les acides chloroacétiques, l'acide oxalique et l'acide éthylhexanoïque, et comme acide de Lewis il est possible d'utiliser une ou plusieurs substances du groupe comprenant le bromure de magnésium, le chlorure de zinc, le bromure de zinc, le chlorure d'aluminium, le bromure d'aluminium, le tétrachlorure de titane, le tétrachlorure d'étain et le trifluorure de bore.